# EUROPEAN PATENT SPECIFICATION

(11) **EP 2 303 450 B1**
(45) Date of publication and mention of the grant of the patent: **30.04.2014**
(21) Application number: 09726149.9
(22) Date of filing: 23.03.2009
(51) Int. Cl.: A61L 9/16, B01J 20/28, B01D 46/00, A61L 9/01

(54) **USE OF A HAZARDOUS SUBSTANCE-REMOVING MATERIAL**
VERWENDUNG EINES MATERIALS ZUR ENTFERNUNG VON GEFAHRSTOFFEN
UTILISATION D' UN MATÉRIAU D ÉLIMINATION D UNE SUBSTANCE DANGEREUSE

(30) Priority: 25.03.2008 JP 2008077543
(43) Date of publication of application: 06.04.2011
(73) Proprietor: FUJIFILM Corporation, Tokyo 106-8620 (JP)
(72) Inventor: KOSUGI, Takuji, Ashigarakami-gun Kanagawa 258-8538 (JP)
(74) Representative: HOFFMANN EITLE
(86) International application number: PCT/JP2009/056406
(87) International publication number: WO 2009/119861

(56) References cited:
- EP-A1- 1 607 107
- EP-A1- 1 676 519
- WO-A1-95/05233
- JP-A- 2003 210 558
- JP-A- 2004 065 731
- JP-A- 2006 069 141
- JP-A- 2006 159 133
- JP-A- 2006 181 293
- JP-A- 2007 160 137

## Description

### Technical Field

The present invention relates to the use of a material for removing hazardous substances from gas. More specifically, the present invention relates to the use of a hazardous substance-removing material which affords a high capture rate and which is comprised of an antibody supported by a support.

### Background Art

Antibody-supporting filters are useful as materials that deactivate hazardous substances such as viruses and various bacteria in liquid and gas phases. In an antibody -supporting filter, hazardous substances are first captured by the filter, after which the hazardous substances that have been captured are deactivated by the antibody. Accordingly, the filter serving as the substrate (support) must afford an adequate capture rate for efficient deactivation to occur.

Japanese Patent No. 3642540 discloses a hazardous substance-removing material in which an antibody is supported on a support. Fiber materials are given as examples of the support, but there is no description of means for increasing the capture rate.

Japanese Unexamined Patent Publication (KOKAI) No. 2001-269520 discloses a long-lived electrostatic air filter, combining wool fiber and homogeneous fibers, that leaks little static electricity and in which the capture rate decreases little. However, the wool fiber that is employed presents drawbacks in the form of a high official moisture regain, little resistance to stretching and abrasion, and, as an animal fiber, a propensity to attract insects.

EP-A-1 607 107 teaches a material for removing hazardous substances comprising antibodies supported on a support. The support is formed from fibres such as polyester or nylon. The antibodies may be chicken antibodies.

EP-A-1 676 519 teaches a cleaning tool for wiping an indoor surface. The tool comprises an electrostatic fibrous base material which supports an antigenicity-reducing component which reduces the antigenicity of an allergy-inducing substance.

### Disclosure of the Invention

The object of the present invention is to provide the use of a hazardous substance-removing material comprised of a support on which is supported an antibody, affording a high capture rate and permitting long-term use, in which the antibody is efficiently utilized.

According to a first aspect, the present invention provides the use of a material for removing hazardous substances from gas comprising a layer comprised of negatively charged polyolefin fibers, and a layer comprised of positively charged other fibers which support an antibody, the other fibers being selected from one or more of cellulose ester fibers, acrylic fibers and polyamide fibers.

According to a second aspect, the present invention provides a method for removing hazardous substances from gas by using a material comprising a layer comprised of negatively charged polyolefin fibers, and a layer comprised of positively charged other fibers which support an antibody, the other fibers being selected from one or more of cellulose ester fibers, acrylic fibers and polyamide fibers.

Preferably the official moisture regain of said other fibers is 1% or more and less than 7%.

Preferably the antibody is a chicken egg antibody.

### Best Mode of Carrying Out the Invention

The present invention will be described in greater detail below.

The hazardous substance-removing material for use in the present invention is comprised of a support on which is supported an antibody. To increase the capture rate, a charged substrate is employed as the support, i.e., as the substrate supporting the antibody. The substrate employed in the hazardous substance-removing material of the present invention is comprised of a polyolefin fiber that readily assumes a negative charge, and another fiber positioned to the positive side of the polyolefin fiber in the electrostatic charge table, and can be formed by imparting charges to these fibers. The other fiber may be comprised of one or more fibers, and is desirably comprised of one fiber. The types of fiber and manufacturing method are described further below.

Sheet-like woven cloth or nonwoven cloth is employed in the substrate thus formed. Nonwoven cloth is desirable. Charged nonwoven cloth is commonly referred to as electret nonwoven cloth (or nonwoven cloth electret).

The hazardous substance-removing material for use in the present invention increases the capture rate through the use of an electrostatically charged substrate as the support. The substrate comprises two or more fibers with different positions in the electrostatic charge table. Therefore, when air, for example, passes between the two or more fibers, the friction that is generated naturally produces a charge, the electret effect functions, and the capture rate is maintained.

The antibody is supported by the other fiber (the fiber that develops a positive charge). Generally, materials that are freely floating in the air tend to develop a negative charge. In viruses, which are partially enveloped, the envelope tends to develop a negative charge. Accordingly, supporting the antibody on the fiber that develops a positive charge tends to increase the capture rate while simultaneously causing hazardous substances to be deactivated by the antibody.

The two or more fibers of differing positions in the electrostatic charge table may be superposed in layers of fibers in the form of nonwoven cloth or the like having flat surfaces. These layers can be bonded together so as to be in contact with each other. When the layers are in contact with each other, charging occurs readily due to friction between the two parts that are in contact with each other. The bonding can be accomplished by heating that is employed to form bonding points, described further below, or by processing employing an adhesive or plasticizing solvent. One desirable example is a method employing a hot melt adhesive. It is desirable to bond together only portions that do not effectively function as hazardous substance-removing agents, and to leave effectively functioning portions in a state in which friction constantly occurs. The bonded portions can be, for example, portions along the outer perimeter. The phrase "portions effectively functioning as hazardous substance-removing agents" means portions that support antibody or portions corresponding to portions that support antibody.

The electrostatically charged substrate is prepared (charged) by conducting a charging process based on corona discharge by the usual method for nonwoven cloth comprised of olefin fiber. Examples of the charging method include: electroelectret, thermoelectret, radioelectret, mechanoelectret, photoelectret, and magnetoelectret treatments. Primarily electroelectret and thermoelectret treatments are industrially employed for nonwoven cloth filters. A corona discharge electroelectret treatment is desirably employed. Superposing nonwoven cloths of differing positions in the electrostatic charge table causes interface charge separation to occur due to the polyolefin electret nonwoven cloth alone, and the charge is maintained.

An electrostatic charge table of major substances is provided below for reference.

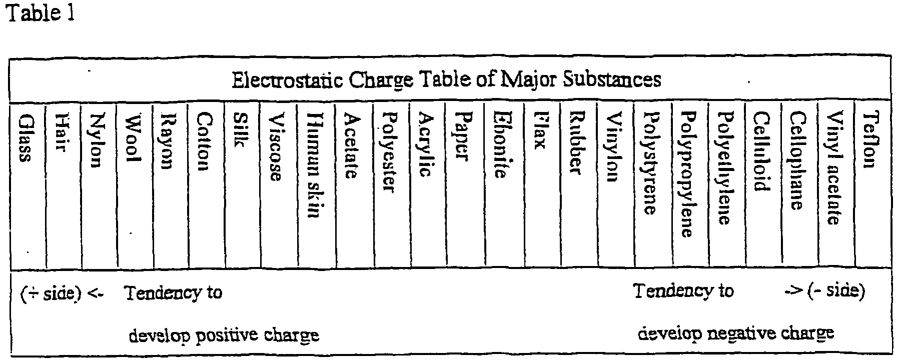

In the hazardous substance-removing material for use in the present invention, a combination of a polyolefin fiber and some other fiber positioned to the positive side of polyolefin fiber in the electrostatic charge table selected from cellulose ester fibers, acrylic fibers and polyamide fibers may be employed as the support.

The selection of such fibers makes it possible to impart a negative charge to the polyolefin fiber and a positive charge to the other fiber by means of the above-charging process.

Examples of polyolefin fibers include polyethylene, polypropylene and polybutene. Among these, polypropylene is particularly preferred. Nonwoven cloth of polyolefin fiber can be manufactured by the meltblown method.

The other fiber functions to maintain the activity of the antibody.

The term "cellulose ester" refers to a cellulose derivative obtained by esterifying a hydroxyl group of cellulose with an organic acid. Examples of an organic acid used for esterification include fatty carboxylic acids such as acetic acid, propionic acid, and butyric acid and aromatic carboxylic acids such as benzoic acid and salicyclic acid. They may be used alone or in combination. The rate of substitution of a hydroxyl group of cellulose with an ester group is not particularly limited; however, it is preferably 60% or more.

As a cellulose ester fiber, a cellulose acylate fiber is preferable. The term "cellulose acylate" used herein refers to cellulose ester in which some or all of hydrogen atoms of a hydroxyl group of cellulose are substituted with an acyl group. Examples of an acyl group include an acetyl group, a propionyl group, and a butylyl group. In terms of structure, a single group among the above examples may be substituted, or two or more acyl groups may be subjected to mixed substitution. The total sum of degrees of acyl group substitution is preferably 2.0 to 3.0, more preferably 2.1 to 2.8, and particularly preferably 2.2 to 2.7. Among them, cellulose acetate, cellulose acetate propionate, or cellulose acetate butylate capable of achieving such degree of substitution is preferable, and cellulose acetate is most preferable. In general, it has been known that a solvent for cellulose acylate varies depending on the degree of esterification. It is also possible to produce a support with cellulose acylate having a high esterification rate in advance and then subject the support to alkali hydrolysis treatment or the like for hydrophilicization of the surface thereof.

According to the present invention, the term "polyamide" refers to a fiber comprising a linear polymer having a chemical structure unit comprising an amide bond.

Among polyamides, a linear aliphatic polyamide, which is a combination of an aliphatic diamine such as ethylenediamine, 1-methylethylenediamine, 1,3-propylenediamine, or hexamethylenediamine and an aliphatic dicarboxylic acid such as malonic acid, succinic acid, or adipic acid, is preferable. Nylon 66 is particularly preferable.

In addition to the above diamine and dicarboxylic acid, aliphatic polyamide comprising a single component or copolymer components selected from among the following examples can be used: lactams such as ε-caprolactam and laurolactam; aminocarboxylic acids such as aminocaproic acid and aminoundecanoicacid; and para-aminomethyl benzoic acid. Nylon 6 produced using ε-caprolactam alone is particularly preferable.

In addition to the above, the following may be used: an aliphatic polyamide in which cycloaliphatic diamine such as cyclohexanediamine, 1,3-bis(aminomethyl)cyclohexane, or 1,4-bis(aminomethyl)cyclohexane is partially or entirely used as a material aliphatic diamine; and/or an aliphatic polyamide in which cycloaliphatic dicarboxylic acid such as 1,4-cyclohexane dicarboxylic acid, hexahydroterephthalic acid, or hexahydroisophthalic acid is partially or entirely used as dicarboxylic acid.

Further, examples of the above polyamide further include a polyamide with decreased water absorbability and an improved elastic modulus in which aromatic diamine such as aliphatic paraxylylene diamine (PXDA) or metaxylylene diamine (MXDA) and aromatic dicarboxylic acid such as terephthalic acid are partially used as starting materials. Moreover, a polymer having a side chain comprising an amide bond such as polyacrylic acid amide, poly(N-methylacrylic acid amide), or poly(N,N-dimethylacrylic acid amide) may be used.

Among polyamides, nylon 66 or nylon 6 is most preferable. This is because the following properties of such polyamide are preferable to be used as the support of the present invention: appropriate hygroscopic properties derived from amide bonds; ease of inducing fiber axis orientation of a molecular chain comprising a long-chain fatty acid having an appropriate length that results in relatively high extensibility; a dynamic and kinetic tendency not to be melted due to high melting temperature and thermal capacity (resistance to melting); flexibility of a molecular chain comprising a long-chain fatty acid; and a tendency to not cause fibrillation or kink band formation (such tendency being imparted as a result of formation of a hydrogen bond between amide bonds), that is to say, repetitive bending and stretching properties.

Likewise, in order to improve strength or dimensional stability, the support can be reinforced with another suitable structural material such as a metal, polymer, or ceramic. The reinforcement material is desirably employed on a portion other than the portion that is essentially the outermost surface of the side on which the hazardous substance-removing material is applied (for example, the reinforcement material is preferably employed on the opposite side, or used as a core material or the like).

According to the present invention, the term "acrylic" refers to a fiber comprising recurring units of an acrylonitrile group (mass percentage: 40% or more). Examples thereof include a homopolymer of acrylonitrile; a copolymer of acrylonitrile and a nonionic monomer such as acrylic ester, methacrylic ester, or vinyl acetate; a copolymer of acrylonitrile and an anionic monomer such as vinylbenzenesulfonate or allylsulfonate; and a copolymer of acrylonitrile and a cationic monomer such as vinylpyridine or methylvinylpyridine. So-called "Pro-mix" fiber formed of acrylonitrile and milk casein is also included in this category.

In general, an acrylic fiber is produced by an organic solvent wet spinning method. In this method, when a spinning stock solution is formed into a coagulated thread in a coagulating bath, water as a coagulant is mixed with the spinning stock solution that is spinning-twisted from a nozzle and a spinning solvent is externally diffused from the spinning-twisted stock solution. At such time, water and an organic solvent (e.g., DMF or DMAc) are mutually diffused such that a polymer deposits, resulting in the formation of a line of coagulated thread having a structure in which many cavities are connected to each other in a net form. In addition, such thread is characterized by deformation of a fiber section caused by volume contraction as a result of diffusion of a solvent into a coagulating bath during coagulation and by formation of concave-convex portions as a result of macrofibril structure formation on the surface thereof. Such fine structure is preferable as a structure of a support used in the present invention in terms of an increase in specific surface area or the ease of antibody loading.

An acrylic fiber varies depending on the composition of a starting material polymer, a spinning method, post-treatment conditions during production, and the like. However, in general, a bulky fiber having appropriate hydrophilicity and high weather resistance can be obtained, which is advantageous.

In order to further improve strength, dimensional stability, and the like, a support may be formed as a mixed fiber of polyolefin fiber or the above other fiber and a fiber other than polyolefin fiber and the above other fiber, such as polyester fiber.

Further, the volume swelling degree in pure water at 20°C of a fiber constituting the support used in the present invention is not less than 1.1% to less than 10%, preferably not less than 1.1% to less than 8%, particularly preferably not less than 1.1% to less than 6%, and most preferably not less than 1.1 % to less than 5%. In addition, the volume swelling degree in pure water at 20°C of a fiber according to the present invention refers to the volume swelling degree which is obtained by measuring the fiber density of each sample in a dried state before immersion of the sample in pure water at 20°C for 1 hour and that after such immersion. Such values are obtained by the density gradient tube method (JIS-K7112).

Regarding mechanical and physical properties and dimensional stability of a fiber constituting a support, the tensile elastic modulus in a dried state is preferably 25% or more. The term "tensile elastic modulus in a dried state" used herein refers to the degree of elongation at break of a fiber in a tensile test at 20°C, provided that such fiber has been dried for a sufficiently long period of time. In general, a fiber having a tensile elastic modulus in a dried state of 10% or more is preferable for processing such as fabric formation. In order to prevent breakage upon filter processing or practical use (such breakage leading to reduction in filtration efficiency), the tensile elastic modulus is preferably 25% or more, more preferably 30% or more, and most preferably 35% or more.

The official moisture regain of the fiber constituting the support is preferably not less than 1.0% to less than 7.0%, more preferably not less than 3.0% to less than 6.5%, most preferably not less than 5.0% to less than 6.5%. In particular, the other fiber (the positively charged fiber) preferably has the official moisture regain of not less than 1.0% to less than 7%, for maintaining mechanical strength of the fiber and the activity of the antibody.

Within the above range of official moisture regain, the expression of the activity of a supported antibody and the mechanical strength, rigidity, dimensional change stability in a use environment (particularly humidity) of a support can be achieved. Further, a filter obtained therewith can exhibit high performance and reliability.

In addition, the term "moisture regain" used herein refers to an official moisture regain. The term "official moisture regain" refers to a moisture regain of a fiber that has been left in an environment at 20°C and at a relative humidity of 65% for long period of time. Moreover, when a fiber is a mixed fiber further containing a different fiber, the term refers to the official moisture regain of the total mixed fibers.

Preferably, the surface of a fiber constituting a support has fine concave-convex portions several tens nanometers to several micrometers in size. The shape of a concave-convex portion may be a three-dimensionally shaped groove or ridge which is formed in the direction parallel to the fiber direction or in the direction vertical to the same, that is to say, in a concentric direction with respect to the fiber axis. Such three-dimensionally shaped groove or ridge may exist at an arbitrary proportion or density, provided that an arbitrary angle is formed in the direction between the parallel direction and the vertical direction to the fiber direction. A sample obtained by a known method for cellulose acetate fiber spinning is known to have a fiber section having a variable chrysanthemum-like shape as a result of skin layer formation on the surface thereof and depression of a skin layer due to solvent drying. In a preferred embodiment, such concave-convex portions are used for the present invention.

The above fine concave-convex portions several tens nanometers to several micrometers in size may have holes and/or projections. Preferably, such holes or projections have an average diameter of 50 nm to 1 µm. For example, such holes and projections can be formed by cavitation of a solution, or they can be formed in a spinning step of a method using a solution in which a fine dispersoid is dispersed (e.g., a mixture containing a slurry in which barium sulfate particles are dispersed), or in a subsequent step by a method involving hydrolysis of an acyl group, surface oxidation treatment, or the like (e.g., the exposure of a cellulose portion on the fiber surface with the use of an alkaline water solution followed by generation of microcraters by an enzyme treatment).

The average fiber diameter of a fiber used for the hazardous substance removing material of the present invention is preferably 50 µm or less, more preferably 10 µm or less, particularly preferably 1 µm or less, and most preferably 100 nm or less. When vinylon is used as the support, the average fiber diameter is preferably 50 µm or less, more preferably 45 µm or less, and most preferably 35 µm or less. The average fiber diameter of the present invention is obtained by measuring the diameters of fibers in arbitrarily selected 300 sites on a scanning electron microscope (SEM) image for observation and averaging the results by calculation.

As to the method for producing the fiber used in the present invention, there are typical production methods such as melting spinning, wet spinning, dry spinning, and dry-wet spinning, and methods in which the fiber is made fine by a physical process (such as strong mechanical shearing using an ultrahigh pressure homogenizer), although dry spinning or dry-wet spinning is preferably employed for obtaining a stable quarity of product. For producing an uniform fiber having an average fiber diameter of 100 nm or less, the electrospinning method disclosed in "Kakou Gijyutsu (Processing Technology)", 2005, Vol. 40, No.2, p.101 and p.167; "Polymer International", 1995, Vol. 36, pp.195-201; "Polymer Preprints", 2000, Vol. 41(2), p.1193; "Journal of Macromolecular Science: Physics", 1997, B36, p.169; and the like is preferably used.

Regarding the solvent used for the spinning, any solvent may be used as long as it dissolves the resin used for synthetic resin fibers. Examples thereof include: chloride-based solvents such as methylene chloride, chloroform, and dichloroethane; amide-based solvents such as dimethylformamide, dimethylacetamide, and N-methylpyrrolidone; ketone-based solvents such as acetone, ethyl methyl ketone, methyl isopropyl ketone, and cyclohexanone; ether-based solvents such as THF and diethyl ether; alcohol-based solvents such as methanol, ethanol, and isopropanol; and water. These solvents may be used either singularly, or in mixtures of a plurality of types thereof.

The resin solution used for the electrospinning method may be added with a salt such as lithium chloride, lithium bromide, potassium chloride, and sodium chloride.

Preferably, fibers constituting a support partially adhere to each other such that a structure forming a three-dimensional network is obtained. The use of such structure results in the improvement of mechanical tolerance upon processing or practical use, leading to the improvement of reliability of the hazardous substance removing material. Further, antibody-supporting properties can be improved. Adhesion between fibers can be observed by a method involving SEM or the like. The density of fiber adhesion points is preferably 10 adhesion points or more in a 1-mm square on the projected surface area of the hazardous substance removing material and preferably 100 adhesion points or more in the same.

Regarding a method for forming adhesion points, adhesion points may be formed by a dry spinning method or by a melt spinning method. After spinning, adhesion point formation treatment may be carried out by heating or adding an adhesive/plasticizing solvent or the like. In view of production cost, it is preferable to form adhesion points by a dry spinning method with the use of an appropriate solution formulation.

A nonwoven cloth that is subjected the above charging process can be prepared in the manner described above.

The antibody used for the hazardous substance removing material is a protein which is reactive (antigen-antibody reaction) specifically to a specific hazardous substance (antigen), has a molecule size of 7 to 8 nm, and is in a Y-shaped molecular form. A pair of branch portions of the antibody in the Y-shaped molecular form is called Fab, and a stem portion thereof is called Fc, among which the Fab portions capture the hazardous substance.

The type of the antibody corresponds to the type of the hazardous substance to be captured. Examples of the hazardous substance to be captured by the antibody include bacteria, fungi, viruses, allergens, and mycoplasmas. Specifically, the bacteria include, for example: the genus *Staphylococcus* (such as *Staphylococcus aureus* and *Staphylococcus epidermidis), Micrococcus, Bacillus anthracis, Bacillus cereus, Bacillus subtilis,* and *Propionibacterium acnes,* as gram-positive bacteria; and *Pseudomonas aeruginosa, Serratia marcescens, Burkholderia cepacia, Streptococcus pneumoniae, Legionella pneumophilia,* and *Mycobacterium tuberculosis,* as gram-negative bacteria. The fungi include, for example, *Aspergillus, Penicillius,* and *Cladosporium.* The viruses include influenza viruses, coronavirus (SARS virus), adenovirus, and rhinovirus. The allergens include pollens, mite allergens, and cat allergens.

Examples of methods for producing the antibody include: a method in which an antigen is administered to an animal such as a goat, a horse, a sheep, and a rabbit, and a polyclonal antibody is refined from the blood thereof; a method in which spleen cells of an animal to which an antigen is administered and cultured cancer cells are subjected to cell fusion and a monoclonal antibody is refined from a culture medium thereof or from a humor (such as ascites) of an animal in which the fussed cells are implanted; a method in which an antibody is refined from a culture medium of genetically modified bacteria, plant cells, or animal cells to which antibody producing gene is introduced; and a method in which a chicken, to which an antigen is administered is allowed to lay an immune egg and a chicken antibody is refined from yolk powder obtained by sterilizing and spray-drying the yolk of the immune egg. Of all the above methods, the method for obtaining the antibody from a chicken egg enables easy mass production of the antibody, reducing the cost of the hazardous substance removing material.

The antibody used for the hazardous substance removing material is preferably a chicken egg antibody.

Regarding methods for immobilizing (fixing) the antibody to the support, there are: other than a method in which the antibody is physically adsorbed to the support, a method in which, after a support is subjected to silane treatment using γ-aminopropyl-triethoxysilane or the like, an aldehyde group is introduced on the surface of the support by glutaraldehyde or the like, to effect a covalent bond between the aldehyde group and an antibody; a method in which an untreated support is immersed into an aqueous solution of an antibody to cause ion boding, thereby immobilizing the antibody to the support; a method in which an aldehyde group is introduced to a support having a specific functional group to effect a covalent bond between the aldehyde group and an antibody; a method in which a support having a specific functional group is ion-bonded to an antibody; and a method in which a support is coated with a polymer having a specific functional group, followed by an introduction of an aldehyde group to effect a covalent bond between the aldehyde group and an antibody.

Here, the specific functional group includes an NHR group (R represents an alkyl group of any of methyl, ethyl, propyl, and butyl except H), an NH₂ group, a C₆H₅NH₂ group, a CHO group, a COOH group, and an OH group.

Moreover, there is also a method in which a functional group on the surface of the support is replaced with another functional group using BMPA (N-β-Meleimidopropionic acid) or the like, to effect a covalent bond between the resultant functional group and an antibody (an SH group is replaced with a COOH group by BMPA).

Further, there is another method in which a molecule (such as an Fc receptor and a protein A/G) which is selectively bindable to the Fc portion of the antibody is introduced on the surface of a support, to cause it to be bonded to the Fc of the antibody. In this case, the Fab for capturing a hazardous substance are arranged outwards from the support to cause increase in contact possibility of the hazardous substance to the Fab, resulting in efficient capturing of the hazardous substance.

The antibody may be supported on the support through a linker. In this case, the degree of freedom of the antibody on the support increases, so that the antibody can readily reach the hazardous substance, attaining a high removal performance. Divalent or multivalent crosslinking reagent may be used as the linker. Specifically, there are listed maleimide, NHS (N-Hydroxysuccinimidyl) ester, imide ester, EDC (1-Ethyl-3-[3-dimetylaminopropyl] carbodiimido), and PMPI (N-[p-Maleimidophenyl]isocyanete), which are selectively or non-selectively bonded to a target functional group (an SH group, an NH₂ group, a COOH group, and an OH group). Moreover, crosslinking agents have different crosslinking distances (spacer arm), and therefore, the distance can be selected within the range between about 0.1 nm to about 3.5 nm according to the target antibody. In view of efficient capturing of the hazardous substance, the linker is preferably bindable to the Fc of the antibody.

Regarding the method for introducing the linker, either one of the following methods may be possible: a method in which a linker is bonded to an antibody, and then the resultant linker is further bonded to the antibody; and a method in which a linker is bonded to a support, and then an antibody is bonded to the linker on the support.

As mentioned above, the antibody is supported by the other fiber (the fiber that develops a positive charge).

The hazardous substance-removing material for use in the present invention removes hazardous substances from a gas phase. The hazardous substance-removing material of the present invention can be employed in air purifier filters or masks.

In the hazardous substance-removing material for use in the present invention, antibody activity is high and a high capture rate can be achieved even in dry environments where the antibodies come into contact with a gas phase.

### Examples

The present invention is described in greater detail below through embodiments. The following embodiments can be suitably modified without departing from the scope of the present invention. Accordingly, the scope of the present invention is not limited to the specific examples given below.

### (Preparation of filter 1)

Polypropylene nonwoven cloth (meltblown method) with a unit weight of 100 g/m² and an average fiber diameter of 12 micrometers as measured by SEM was heated to a temperature of 100°C, thoroughly dried, and charged by being subjected to a corona discharge by the usual method.

Subsequently, immune eggs laid by chickens that had been administered antigen were purified to obtain influenza virus antibody (IgY antibody). This antibody was dissolved in phosphate-buffered saline (PBS) and a solution with an antibody concentration of 100 ppm was prepared. The solution was blown as a mist onto the above-described filter to transfer the antibody to the fiber surface. The filter was then dried overnight at 50°C to obtain filter 1.

### (Preparation of filter 2)

Cellulose acetate nonwoven cloth (meltblown method) with a unit weight of 50 g/m² and an average fiber diameter of 20 micrometers as measured by SEM was loaded with antibody in the same manner as for filter 1. The cellulose acetate nonwoven cloth obtained was thoroughly dried and then bonded along the outer perimeter portion thereof to polypropylene nonwoven cloth 50 g/m² that had been subjected to the same electret treatment as that employed in the manufacturing of filter 1, using a melted polyolefin fiber hot-melt adhesive (Moresco Melt, made by Matsumura Oil Co., Ltd.), to obtain filter 2.

### (Preparation of filter 3)

With the exceptions that polyethylene nonwoven cloth that had been subjected to an electret treatment, had a unit weight of 50 g/m², and had an average fiber diameter of 12 micrometers as measured by SEM, and acrylic nonwoven cloth (meltblown method) with a unit weight of 50 g/m² and an average fiber diameter of 21 micrometers as measured by SEM were employed, filter 3 was prepared in the same manner as filter 2.

### (Preparation of filter 4)

With the exception that the cellulose acetate nonwoven cloth was replaced with nylon nonwoven cloth (meltblown method) with a unit weight of 50 g/m² and an average fiber diameter of 21 micrometers as measured by SEM, filter 4 was prepared in the same manner as that of filter 2.

### (Preparation of filter 5)

With the exception that cellulose acetate nonwoven cloth (meltblown method) having a unit weight of 100 g/m² and an average fiber diameter of 20 micrometers as measured by SEM was employed instead of propylene nonwoven cloth, filter 5 was prepared in the same manner as that of filter 1.

### (Preparation of filter 6)

With the exception that cellulose nonwoven cloth (meltblown method) having a unit weight of 50 g/m² and an average fiber diameter of 10 micrometers as measured by SEM was employed instead of cellulose acetate nonwoven cloth, filter 6 was prepared in the same manner as filter 2. The cellulose was imparted with a positive charge relative to polypropylene.

### <Evaluation of the virus deactivation rate>

The virus deactivation rates of antibody filters 1 to 4 were evaluated.

Purified influenza virus that had been diluted 10-fold with PBS (virus concentration of 200,000 plaques/mL) was employed as the test virus solution. Each of the above filters was cut into a 5 cm square and mounted securely in the center of a virus nebulizing test device. Sample virus solution was placed in a nebulizer positioned upstream, and a virus recovery device was mounted downstream. Compressed air was delivered by an air compressor and test virus solution was sprayed as a mist from the spray nozzle of the nebulizer. A gelatin filter was positioned downstream from a mask, air was drawn from within the test device for five minutes at a flow rate of 10 L/minute, and the passing virus mist was captured.

Following the test, the gelatin filter that had captured the virus was recovered, and the viral infection value following passage of the sample was determined by the TCID 50 method (a method that measures the quantity required to infect 50 percent of cells) with MDCK cells. The one-pass viral removal rate of each of the filters was calculated by comparing the viral infection value of the gelatin filter when the sample was present and when it was absent. The results are given in Table 2.

### <Capture performance test>

The above filters were punched into 150 mm squares, set into sample holders, and placed within test ducts of identical diameter. The particle trapping rate test described in the dust mask standards established based on Article 42 of the Industrial Safety and Health Law was implemented and the capture rate and the final capture rate following the accumulation of 100 mg of NACL were measured. The results are given in Table 2.

### <Measurement of moisture content>

Prior to combining the filters that had been prepared, each of the nonwoven cloth samples was placed for a week or more in an environment of 20°C and 65 percent relative humidity. The moisture content of each sample was then measured with a halogen hygrometer MB35 (made by Ohaus Corporation). The results are given in Table 2.

**Table 2**

| Filter | Virus deactivation rate | Moisture content | Capture rate | Final capture rate | Remarks |
|---|---|---|---|---|---|
| | (%) | (%) | (%) | (%) | |
| 1. Polypropylene | 10 | 0.4 | 70 | 30 | Comp. Ex. |
| 2. Polypropylene/acetate | 90 | 0.4/6.3 | 85 | 84 | Invention |
| 3. Polyethylene/acrylic | 85 | 0.4/1.9 | 85 | 84 | Invention |
| 4. Polypropylene/nylon | 90 | 0.4/4.3 | 90 | 90 | Invention |
| 5. Acetate | 75 | 6.3 | 40 | 20 | Comp. Ex. |
| 6. Polypropylene/cellulose | 11 | 0.4/11.2 | 80 | 60 | Invention |

As will be clear from Table 2, the capture rate of the hazardous substance-removing material was increased by static electricity generated by friction between the polyolefin fiber and the fiber positioned to the positive side of polyolefin fiber in the electrostatic charge table. Further, the virus deactivation rate was high when the moisture content of the positively charged fiber was from 1 to 7 percent.

### Industrial Applicability

The present invention provides the use of a material for removing a hazardous substance from gas. The material comprises a support on which is supported an antibody, affording a high capture rate, in which the antibody is efficiently utilized. The capture rate can be extended in normal use, making it possible to employ the hazardous substance-removing material for extended periods.

## Claims

1. The use of a material for removing hazardous substances from gas comprising:
a layer comprised of negatively charged polyolefin fibers, and
a layer comprised of positively charged other fibers which support an antibody, the other fibers being selected from one or more of cellulose ester fibers, acrylic fibers and polyamide fibers.

2. The use according to Claim 1, wherein the official moisture regain of said other fibers is 1 percent or more and less than 7 percent.

3. The use according to Claim 1 or Claim 2, wherein said antibody is a chicken egg antibody.

4. A method for removing hazardous substances from gas by using a material comprising:
a layer comprised of negatively charged polyolefin fibers, and
a layer comprised of positively charged other fibers which support an antibody, the other fibers being selected from one or more of cellulose ester fibers, acrylic fibers and polyamide fibers.

5. A method according to Claim 4, wherein the official moisture regain of said other fiber is 1 percent or more and less than 7 percent.

6. A method according to Claim 4 or Claim 5, wherein said antibody is a chicken egg antibody.

## Patentansprüche

1. Verwendung eines Materials zum Entfernen von schädlichen Substanzen aus Gas, umfassend:
eine Schicht, die negativ geladene Polyolefinfasern umfasst, und
eine Schicht, die positiv geladene, andere Fasern umfasst, welche einen Antikörper trägern, worin die anderen Fasern ausgewählt sind aus einem oder mehr von Celluloseesterfasern, Acrylfasern und Polyamidfasern.

2. Verwendung gemäß Anspruch 1, worin die offizielle Feuchtigkeitsaufnahme der anderen Fasern 1 % oder mehr und weniger als 7 % beträgt.

3. Verwendung gemäß Anspruch 1 oder Anspruch 2, worin der Antikörper ein Hühnerei-Antikörper ist.

4. Verfahren zum Entfernen von schädlichen Substanzen aus Gas unter Verwendung eines Materials, das umfasst:
eine Schicht, die negativ geladene Polyolefinfasern umfasst, und
eine Schicht, die positiv geladene, andere Fasern umfasst, die einen Antikörper trägern, worin die anderen Fasern ausgewählt sind aus einem oder mehr von Celluloseesterfasern, Acrylfasern und Polyamidfasern.

5. Verfahren gemäß Anspruch 4, worin die offizielle Feuchtigkeitsaufnahme der anderen Fasern 1 % oder mehr und weniger als 7 % beträgt.

6. Verfahren gemäß Anspruch 4 oder Anspruch 5, worin der Antikörper ein Hühnerei-Antikörper ist.

## Revendications

1. Utilisation d'un matériau pour éliminer des substances dangereuses d'un gaz comprenant :
une couche constituée de fibres de polyoléfine négativement chargées, et
une couche constituée d'autres fibres positivement chargées qui supportent un anticorps, les autres fibres étant choisies parmi une ou plusieurs parmi des fibres d'ester de cellulose, des fibres acryliques et des fibres de polyamide.

2. Utilisation selon la revendication 1, dans laquelle la récupération d'humidité officinale desdites autres fibres est de 1 pourcent ou supérieure et inférieure à 7 pourcents.

3. Utilisation selon la revendication 1 ou la revendication 2, dans laquelle ledit anticorps est un anticorps d'oeuf de poule.

4. Procédé pour l'élimination de substances dangereuses d'un gaz en utilisant un matériau comprenant :
une couche constituée de fibres de polyoléfine négativement chargées, et
une couche constituée d'autres fibres positivement chargées qui supporte un anticorps, les autres fibres étant choisies parmi une ou plusieurs parmi des fibres d'ester de cellulose, des fibres acryliques et des fibres de polyamide.

5. Procédé selon la revendication 4, dans lequel la récupération d'humidité officinale de ladite autre fibre est de 1 pourcent ou supérieure et inférieure à 7 pourcents.

6. Procédé selon la revendication 4 ou la revendication 5, dans lequel ledit anticorps est un anticorps d'oeuf de poule.
